# EUROPEAN PATENT SPECIFICATION

(11) **EP 1 910 799 B1**
(45) Date of publication and mention of the grant of the patent: **23.03.2011**
(21) Application number: 06800723.6
(22) Date of filing: 02.08.2006
(51) Int. Cl.: G01N 33/543, G01N 1/02, C12M 1/30, B01L 3/00

(54) **APPARATUS AND METHOD FOR DETECTING AN ANALYTE**
VORRICHTUNG UND VERFAHREN ZUM NACHWEIS EINES ANALYTS
APPAREIL ET PROCÉDÉ DE DÉTECTION D' UNE ANALYTE

(30) Priority: 02.08.2005 US 705089 P
(43) Date of publication of application: 16.04.2008
(60) Divisional application: 10185068.3
(73) Proprietor: 3M Innovative Properties Company, St. Paul, MN 55133-3427 (US)
(72) Inventor: BOMMARITO, G. Marco, Saint Paul, Minnesota 55133-3427 (US); BURTON, Scott A., Saint Paul, Minnesota 55133-3427 (US); DODGE, Larry H., Saint Paul, Minnesota 55133-3427 (US); GONZALEZ, Bernard A., Saint Paul, Minnesota 55133-3427 (US); SMITH, Jeffrey D., Saint Paul, Minnesota 55133-3427 (US)
(74) Representative: Vossius & Partner
(86) International application number: PCT/US2006/030338
(87) International publication number: WO 2007/016691

(56) References cited:
- WO-A-93/12421
- WO-A-96/14570
- WO-A-98/39657
- WO-A-2004/057331
- US-A- 5 658 531

## Description

### BACKGROUND

Many industries, such as the medical and food service industries, often require the testing of a sample of material in order to determine whether a certain biological bacterium or other organism is present. The presence of such an organism may be indicative of a problem. For example, the presence of the organism may indicate the presence of infection in a person or the presence of a contaminant in food or on a food preparation surface.

In existing methods of testing the sample of material, a sample acquisition device, such as a swab, which includes a porous medium on the end of a shaft, may be used to gather the sample of material. Specifically, the porous medium of the swab may be placed in contact with a sample source, such as a nose, ear, throat, or wound of a person, or a food preparation surface, and a sample may then adhere to the porous medium. Thereafter, the sample acquisition device may be transferred to a different location, such as a laboratory, where the collected sample is transferred from the sample acquisition device to a slide or other external laboratory apparatus in order to run an assay to analyze whether the particular organism of interest is present. The particular organism of interest may be referred to as an "analyte".

In addition to a delay in time, the transfer of the sample acquisition device from the sample source to the off-site location may cause the collected sample to become contaminated or dry out, which may decrease the reliability of the analyte detection. Furthermore, a non-self contained testing device or method may be problematic because the lab technician may be exposed to the analyte during the testing process. The present invention addresses these and/or other problems and provides advantages over other devices, e.g. those described in US 5,658,531. In particular, these problems are solved by the apparatuses and the method as defined in the claims.

### BRIEF SUMMARY

The application discloses, in one aspect, an apparatus for processing a biological material. In illustrative configurations, the apparatus includes a chamber configured to receive an eluted sample. The sample is tested using a testing device separate from the chamber. As disclosed, the sample is introduced into the chamber while the apparatus is in a first orientation. The orientation of the apparatus is changed to move the eluted sample from the chamber for testing using the testing device.

In an aspect according to the present invention, an apparatus is disclosed for detecting an analyte in a sample of material. The apparatus comprises a housing including an opening configured to receive a sample acquisition device comprising a sample of material, a chamber configured to receive an eluted sample from the sample acquisition device, a testing chamber including a testing device adapted to detect the analyte, and a continuously unobstructed passageway connecting the chamber configured to receive the eluted sample with the testing chamber and being oriented to provide fluid flow from the first chamber to the testing chamber when the apparatus is in a first orientation and to restrict fluid flow to the testing chamber when the apparatus is in another orientation, wherein the testing device comprises a colorimetric sensor for providing a visual indicium of a test result; wherein the first chamber comprises a reagent disposed therein: wherein the testing chamber is in fluidic connection with the chamber configured to receive a sample: wherein the testing chamber is between the chamber configured to receive the eluted sample and a chamber having an absorbent material..

In another illustrative aspect, an apparatus is disclosed for processing a sample of material. The apparatus comprises a base having an opening at a first end adapt to receive a sample acquisition device and a chamber spaced from the opening, and a removable indicator cap including a testing device coupleable to the opening at the first end of the base to test an analyte in the sample of material in the chamber.

In another aspect according to the present invention, a method is disclosed of detecting an analyte in a sample of material. The method comprises eluting a sample of material from a sample acquisition device into a chamber configured to receive an eluted sample in an apparatus according to the present invention, combining the eluted sample with a reagent, and rotating the apparatus at least ninety degrees and testing the eluted sample using a testing device.

In another illustrative aspect, a method is disclosed of detecting an analyte in a sample of material. The method comprises eluting the sample of material from a sample acquisition device at a first end of an apparatus to form an eluted sample, removing the sample acquisition device from the apparatus, attaching a testing device to an opening at the first end of the apparatus, and manipulating the apparatus to cause the eluted sample to contact the testing device.

The above summary is not intended to describe each disclosed embodiment or every implementation of the present invention. The figures and the detailed description which follow more particularly exemplify illustrative embodiments.

### BRIEF DESCRIPTION OF THE DRAWINGS

The present invention will be further explained with reference to the drawing figures listed below, where like structure is referenced by like numerals throughout the several views.
FIG. 1 is a side view of a first exemplary embodiment of an apparatus of the present invention in a sample preparation orientation, where a sample acquisition assembly has been introduced into the apparatus.
FIG. 2 is a side view of an alternate embodiment of a fluid reservoir shown in FIG. 1.
FIG. 3A is a side view of the apparatus of FIG. 1, where the apparatus is in a testing orientation.
FIG. 3B is a schematic of an apparatus that is configured to receive two sample acquisition assemblies.
FIG. 4 is a side view of a second exemplary configuration of an apparatus, where the apparatus is an assembly including a base that is configured to interchangeably receive a sample acquisition assembly (which is introduced into the base) and an indicator cap.
FIG. 5 is a side view of the apparatus of FIG. 4, where a deformable squeeze bulb has been substituted for the syringe-like fluid reservoir shown in FIG. 4.
FIG. 6 is a side view of the apparatus of FIG. 4, where the fluid retained in the fluid reservoir has been released therefrom to render an eluted sample, where the eluted sample is retained in the base.
FIG. 7 is a side view of the apparatus of FIG. 6, where the sample acquisition assembly has been removed from an opening in the base and an indicator cap is aligned to fit in the opening in the base.
FIG. 8 is a side view of the apparatus of FIG. 7, where the indicator cap has been fully introduced into the opening in the base, and where the base now in its testing orientation.
FIG. 9 is a view of the underside of the indicator cap of FIGS. 4, 7, and 8, where a window and color-code diagram are positioned on the underside.

While the above-identified figures set forth several exemplary embodiments of the present invention, other embodiments are also within the invention. In all cases, this disclosure presents the invention by way of representation and not limitation. It should be understood that numerous other modifications and embodiments can be devised by those skilled in the art, which fall within the scope and spirit of the principles of the invention.

### DETAILED DESCRIPTION

The present invention is an apparatus for detecting an analyte, such as Gram-positive or Gram-negative bacteria, in a sample of material. In the embodiments shown, the apparatus includes a chamber configured to receive the sample of material and a testing device adapted to detect an analyte in the sample of material and the apparatus is moveable between a sample preparation orientation and a testing orientation. The apparatus may be moved between the two orientations by an apparatus operator or a mechanical (or electromechanical) machine. In a sample preparation orientation, the sample of material is prepared for detection by the testing device. For example, the sample may be eluted from a sample acquisition device and/or pretreated for detection. Different analytes may require different pretreatments to change the physical or chemical properties of a sample or analyte before analyte detection, and the relevant pretreatments may be incorporated into the present invention. In the exemplary embodiments, the pretreatment includes mixing an eluted sample with a reagent, where the reagent is adapted to react with an analyte that is present in the eluted sample. In the testing orientation, the apparatus is orientated to change a fluid flow path through the apparatus, so that the eluted and/or pretreated sample retained in the chamber of the apparatus contacts the testing device.

By moving the apparatus from the sample preparation orientation to the testing orientation, a flow path through the apparatus is modified, and as a result, different portions of the apparatus are fluidically connected with one another. The chamber is configured so that in the sample preparation orientation, the sample of material (e.g., a sample eluted from a sample acquisition device) retained in the chamber does not contact the testing device. The different apparatus orientations allow an apparatus operator to control the duration of the sample preparation stage as well as the initiation of the testing stage. Specifically, the testing stage does not begin until the operator moves the apparatus from the sample preparation orientation to the testing orientation. Both the sample preparation and testing stages will be described in further detail below.

The inventive apparatus assembly is a relatively simple device that allows a sample of material to be tested for an analyte at or near the sample source. Rather than transferring the sample of material to an off-site laboratory, the present invention allows an operator to obtain a sample of material from a sample source and then test the sample for the presence of an analyte at or near the sample source. This helps to decrease the waiting time for a test result as well as helps to decrease the chances that the sample of material will become contaminated or dried out prior to testing. Further, the sample acquisition device containing the sample of material may be directly introduced into an apparatus of the present invention. This may decrease operator exposure to the sample of material, which may contain a hazardous analyte. In some embodiments, the apparatus is disposable, which helps to provide a clean, if not sterile, apparatus for each use.

In some embodiments, the apparatus of the present invention is configured to receive two or more samples of material and simultaneously (or essentially simultaneously) detect an analyte in each sample. The samples may be from the same sample source or from different sample sources, and the analyte in each sample may or may not be the same analyte. For example, in one embodiment, an apparatus is configured to receive two samples of material, and simultaneously detect Gram-positive bacteria in one sample and Gram-negative bacteria in the second sample. In other embodiments, the apparatus may be configured to receive a single sample of material and simultaneously (or essentially simultaneously) detect two different analytes in the single sample of material, such as by using two different testing devices. For example, in one embodiment, an apparatus is configured to receive a single sample acquisition device, which includes a sample of material, and the apparatus is configured to elute the sample from the sample acquisition device, where two testing devices simultaneously detect Gram-positive bacteria and Gram-negative bacteria in the eluted sample. In this way, the present invention provides a Gram-positive and a Gram-negative test in one apparatus.

The present invention is described in reference to exemplary embodiments, which both use an indirect assay to detect an analyte in a sample of material. A general understanding of the assay process that is used with the exemplary embodiment will help aid in the description of the inventive apparatus. However, the following description of the assay process is not intended to limit the present invention in any way. Rather, the inventive apparatus and method of detecting an analyte in a sample of material may be applied to many different types of assays, direct or indirect.

In accordance with the exemplary embodiments, a sample of material is obtained with a sample acquisition device. Prior to running the assay, the sample of material is prepared. In the sample preparation stage, the apparatus (or a component of the apparatus) is in a sample preparation orientation. The sample of material is eluted (or "released" or "washed") from the sample acquisition device with a first buffer solution, rendering an eluted sample. Hereinafter, "eluted sample" refers to a combination of the sample that is removed from the sample acquisition device and the first buffer solution. Examples of suitable buffer solutions include, but are not limited to, water, physiological saline, pH buffered solutions, or any other solutions or combinations of solutions that elute an analyte from the sample acquisition device. The buffer solution preferably does not interfere with the assay.

The eluted sample is then mixed with a reagent that is adapted to react with (e.g., bind with) any analyte that is present in the eluted sample. If a direct assay is used, a reagent may not be necessary. After the analyte and reagent react, and after a sufficient "reaction time", the apparatus may be moved from its sample preparation orientation to its testing orientation. In the testing orientation, the eluted sample and reagent mixture contact a testing device. In an indirect assay, the testing device detects the presence of a reagent adapted to react with the analyte rather than the analyte itself. Specifically, the reagent and analyte react, and then any remaining reagent (i.e., the reagent that has not reacted with the analyte to form a separate product) reacts with the testing device. For example, any remaining reagent ("first reagent") may react with a second reagent that is immobilized on the testing device. Thereafter, the testing device provides a visual indicium of the presence and/or quantity of reagent. In the embodiment where the reagent reacts with a second reagent on the testing device, the amount of the first reagent that binds with the second reagent is indicated by a color change. It is preferred that the analyte and reagent are given sufficient time to react prior to contacting the testing device. The testing device may be any suitable device, such as a colorimetric sensor.

In one embodiment, the reagent reacts with a surface of the testing device (originally a blue color), and the testing device changes color as the reagent reacts with the testing device. If a large quantity of reagent reacts with the testing device, the testing device may change color from blue to red. If a small quantity of reagent reacts with the testing device, the testing device may not change color and remain blue. The testing device may also be configured to provide an indicium of the quantity of reagent present (which typically represents the quantity of analyte present in the sample of material). For example, the testing device may change color, where the intensity or hue of the color changes depending upon the amount of reagent present. In alternate embodiments, the testing device measures and indicates the amount of reagent in another suitable way.

The quantity of reagent present indicates the quantity of analyte present because typically, a large quantity of reagent present after the reaction with the analyte indicates that there was not a large quantity of analyte present in the sample of material. Similarly, a small quantity of reagent present after the reaction with the analyte indicates that there was a large quantity of analyte present in the sample of material.

In alternate embodiments, at least some of the analyte may be isolated from the eluted sample with a capture medium prior to contacting the analyte with the testing device. It may be desirable to isolate and, in some sense, concentrate the analyte because some analytes are only detected in large quantities. The isolation/concentration may increase the chance of an accurate detection.

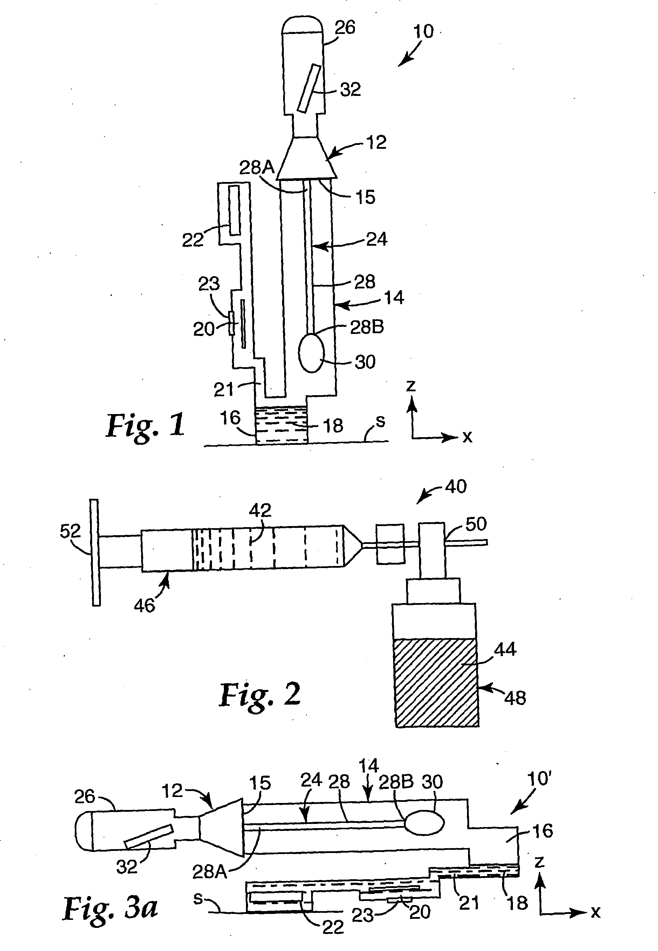

In a first exemplary embodiment, the apparatus is substantially self-contained because generally all the chemistry for detecting the analyte is contained in the apparatus. This decreases the chance that an apparatus operator will be exposed to the analyte and/or fluids that are used in the testing process, such as by an accidental spill or otherwise. The first exemplary apparatus is moved from its sample preparation orientation to its testing orientation by tipping the apparatus over onto its side, about 90 degrees (°). The first exemplary apparatus includes a chamber for receiving a sample of material and a testing device adapted to detect an analyte. The chamber and testing device are configured in a generally side-by-side orientation so that two fluid flow paths are created through an interior of the apparatus. The first fluid flow path in the sample preparation orientation is separate from a second fluid flow path in the testing orientation. In the sample preparation orientation, the sample is blocked from the second fluid flow path, and so, in a sense, the sample is blocked from contacting the testing device. The first exemplary apparatus is described in reference to FIG. 1.

In a second exemplary embodiment, the apparatus is an apparatus assembly that includes a base, which is configured to interchangeably receive the sample acquisition assembly and an indicator cap. The base includes a chamber for receiving an eluted sample of material. The sample acquisition assembly includes a hollow shaft including a distal end and a proximal end, where a porous medium is attached to the distal end and a fluid reservoir is attached to the proximal end. The indicator cap includes a testing device configured to detect an analyte and provide an indicium of the test result. In the sample preparation orientation, a sample acquisition assembly is removably attached to the base, which is positioned below the sample acquisition assembly (i.e., the base has the lowest z-coordinate, where orthogonal x-y-z coordinates are shown in FIG. 1). "Removably attached" indicates that the sample acquisition assembly is not permanently attached to the base. The base is configured to support the sample acquisition assembly and the fluidic reservoir of the sample acquisition assembly is configured to be in fluidic communication with a chamber in the base.

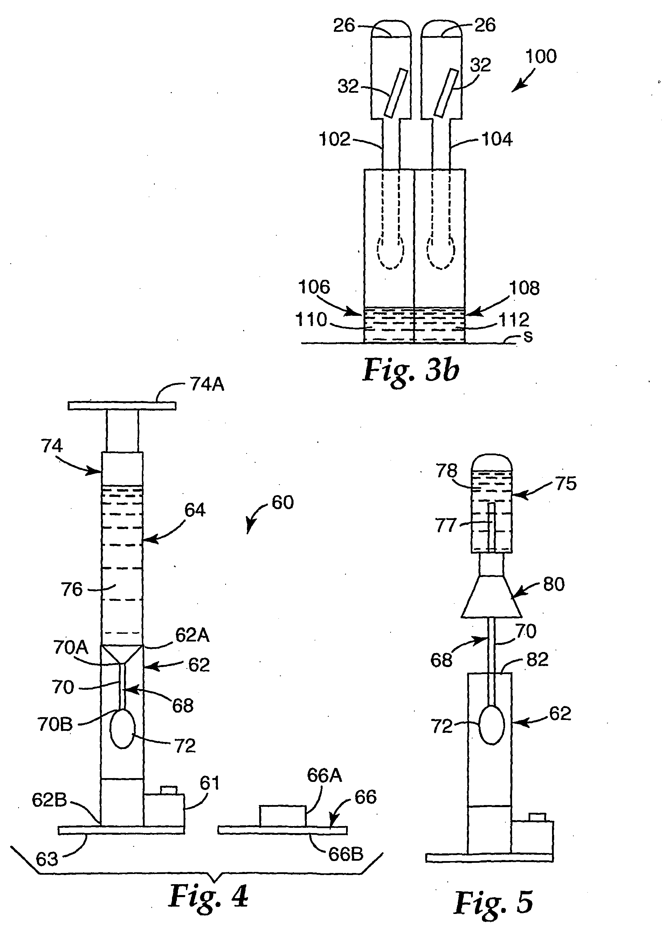

When the base is positioned below the sample acquisition assembly, a first fluid flow path is created from the sample acquisition assembly to the base (where gravity helps to determine the flow path). During this sample preparation stage, the sample is eluted from the sample acquisition device. While still in the sample preparation orientation, the sample acquisition assembly is removed from the base and the indicator cap is removably attached to the base. In the testing orientation, the indicator cap and base are rotated about 180° so that the indicator cap is positioned below the base and a second fluid flow path is created from the base to the indicator cap. The eluted and/or pretreated solution moves along the second fluid flow path to contact the testing device in the indicator cap. The second exemplary embodiment is discussed in further detail in reference to FIG. 4.

FIG. 1 is a side view of the first exemplary embodiment of apparatus 10 of the present invention in a sample preparation orientation, where sample acquisition assembly 12 has been introduced into apparatus 10. Apparatus 10 includes a sample preparation orientation (shown in FIG. 1) and a testing orientation (shown in FIG. 3A). First, in the sample preparation orientation, apparatus 10 is in a generally upright orientation relative to generally horizontal surfaces (e.g., surface s in FIG. 1) on which apparatus 10 is placed. Second, in the testing orientation, apparatus 10 is rotated about 90° in order to alter a fluid flow path through apparatus 10. The testing orientation is shown and described in reference to FIG. 3A.

Apparatus includes housing 14, which is configured to receive sample acquisition assembly 12. Housing 14 further includes chamber 16 for receiving eluted sample 18 (which is a sample of material that has been eluted from sample acquisition assembly 12), testing device 20, channel 21, and absorbent material 22. Testing device 20 is adapted to detect an analyte and provide an output of a test result. Channel 21 provides a passageway to fluidically connect chamber 16 with testing device 20. Absorbent material 22 is positioned adjacent testing device 20 (where testing device 20 is positioned between channel 21 and absorbent material 22). The absorbent material 22 is provided to help the fluid flow past testing device 20 by creating a wicking action.

Sample acquisition assembly 12 is received in opening 15 of housing 14 and is preferably in close conforming contact with opening 15 so that opening 15 is substantially covered by sample acquisition assembly 12. This helps to ensure that apparatus 10 is generally self-contained. Sample acquisition assembly 12 includes sample acquisition device 24 and fluid reservoir 26. Sample acquisition device 24 may be any suitable device, such as a swab. Examples of suitable sample acquisition devices are described in U.S. Patent No. 5,266,266, entitled, "SPECIMEN TEST UNIT", and U.S. Patent Application Serial No. 60/705,140, entitled, "APPARATUS AND METHOD FOR COLLECTING A SAMPLE OF MATERIAL," (Attorney Docket No. 61097US002) which was filed on the same date as the present application.

In the exemplary embodiment, it is preferred that sample acquisition device 24 include hollow shaft 28, having proximal end 28A and distal end 28B, and porous medium 30 attached to the hollow shaft 28 proximate to the distal end 28B. Porous medium 30 of sample acquisition device 24 may be placed in contact with a sample source, such as a nose, ear, or throat of a person (or a wound or other bodily area of interest), or a food preparation surface, and a sample from the sample source may then adhere to porous medium 30. By introducing sample acquisition device 24 (as a part of sample acquisition assembly 12) into opening 15, a sample is introduced into apparatus 10.

The exemplary fluid reservoir 26 retains a fluid (not shown in FIG. 1), which may be a buffer solution. Examples of suitable fluid reservoirs include, but are not limited to, a deformable squeeze bulb, a syringe, or an accordion pleat bulb. An example of a suitable syringe includes a BD HyPak syringe, which is made commercially available by Becton Dickinson, Franklin Lakes, New Jersey. In FIG. 1, fluid reservoir 26 is shown to be a deformable squeeze bulb with snap valve 32 (which has been broken). The type of buffer solution that is to be incorporated into the assay is dependent upon many factors, including the analyte that apparatus 10 is configured to detect. Fluid reservoir 26 is positioned to be in selective fluidic communication with proximal end 28A of hollow shaft 28. "Selective fluidic communication" indicates that there is a valve, plunger (such as in a syringe) or other apparatus operator-activated means of introducing the fluid disposed in fluid reservoir 26 into hollow shaft 28 of sample acquisition device 24. Releasing the fluid into hollow shaft 28 of sample acquisition device 24 elutes a sample adhered to porous medium 30, rendering an eluted sample.

When apparatus 10 is in its sample preparation orientation (FIG. 1), a sample of material is prepared for detection. In accordance with the exemplary embodiment of the present invention, a sample is introduced into apparatus 10 when apparatus 10 is in its sample preparation orientation. Specifically, sample acquisition device 24, which has collected a sample with porous medium 30 is introduced into opening 15 of housing 14. The sample is then eluted from porous medium 30 of sample acquisition device 24 by releasing a fluid retained in fluid reservoir 26. The resulting eluted sample 18 (shown to be retained in chamber 16 in FIG. 1) flows through apparatus along a first flow path, which results when apparatus 10 is in its sample preparation orientation. The first flow path is formed from the sample acquisition device 24 to chamber 16. Therefore, sample acquisition device 24 and chamber 26 are in fluidic communication when apparatus 10 is in its sample preparation orientation.

In the first exemplary embodiment, any analyte that is present in eluted sample 18 is placed in contact with a reagent adapted to react with the analyte. The reagent is preferably dehydrated in order to keep the reagent stable during storage of apparatus 10. In the exemplary embodiment, a dehydrated reagent is disposed within chamber 16 and reconstituted in chamber 16. Upon contact with the dehydrated reagent in chamber 16, eluted sample 18 reconstitutes the reagent in order to reactivate it. The reagent and analyte (if any) that is present in the eluted sample react in chamber 16. An operator may accelerate or otherwise aid the reaction process by agitating apparatus 10, such as by shaking apparatus 10 or vortex mixing the contents of chamber 16.

In alternate embodiments, the dehydrated reagent may be disposed within any suitable place within apparatus. For example, the dehydrated reagent may be disposed in housing 14 upstream of chamber 16 (i.e., along the first flow path before chamber 16). Alternatively, the reagent may be reconstituted outside apparatus 10 and injected into apparatus 10 through hollow shaft 28 of sample acquisition device 24.

FIG. 2 illustrates a dual chamber assembly 40 that allows a fluid 42 and a reagent 44 to be kept separate until an operator initiates the mixing process. The dual chamber assembly 40 provides an alternate embodiment for providing fluid 26 to elute a sample of material from a sample acquisition device 24. As shown, the dual chamber assembly 40 includes syringe 46, which retains fluid 42, and a reagent compartment 48. An adaptor 50 as shown is attached to reagent compartment 48 and syringe 46 to fluidly connect reagent compartment 48 and syringe 46. Syringe 46 includes a plunger member 52. The plunger member 52 of syringe 46 is depressed so that fluid 42 flows from syringe 46 to reagent compartment 48. Reagent compartment 48 may then be agitated to aid the dehydrated reagent 44 in dissolving. After dehydrated reagent 44 has dissolved in fluid 42, the contents of reagent compartment 48 may be withdrawn into syringe 46 with the aid of a vacuum-action actuated by plunger 52. Thereafter, reagent compartment 48 and adaptor 50 may be removed from the syringe 46 and the contents of syringe 46 (i.e., the reagent and fluid solution) may be introduced into hollow shaft 28 of sample acquisition device 24.

In general, the location of the reagent and analyte reaction depends on where the reagent is disposed. However, it is preferred that the analyte react with a reagent when apparatus 10 is in its sample preparation orientation because as previously stated, in an indirect assay, it is the reagent that reacts with the testing device.

After sufficient time to allow reagent and any analyte present in eluted sample 18 to react, apparatus 10 may be moved from its sample preparation orientation (FIG. 1) to its testing orientation (FIG. 3A): FIG. 3A is a side view of the first exemplary embodiment of apparatus 10 in its testing orientation. Apparatus 10 has been rotated about 90° (where a reference point is provided by orthogonal x-z coordinates in FIGS. 1 and 3) from its sample preparation orientation to its testing orientation.

In the testing orientation of apparatus 10, chamber 16, channel 21, and testing device 20 are configured to have different z-coordinates, where chamber 16 has the greatest z-coordinate and testing device has a smallest z-coordinate. As a result of the gradual downward slope from chamber 16 to testing device 20, a second flow path is formed from chamber 16 to testing device 20. In the exemplary embodiment, eluted sample 18 flows from chamber 16 to testing device 20 with the aid of gravity. As previously discussed, absorbent material 22 may also help encourage fluid flow past testing device 20. In alternate embodiments, other means of encouraging eluted sample 18 to flow from chamber 16 to testing device 20 are used.

After apparatus 10 is moved to its testing orientation 10, eluted sample 18 flows along the second flow path from chamber 16, through channel 21, and contacts testing device 20. In the first exemplary embodiment, channel 21 includes microfluidic elements for controlling the flow of fluid from chamber 16 to testing device 20. Testing device 20 may require fluid to flow past it at or below a certain rate in order for the analyte or reagent in the fluid to react with testing device 20. In the case of the exemplary embodiment, an indirect assay is used, and so it is the reagent in the fluid that reacts with testing device 20. A plurality of microfluidic elements may help regulate this fluid flow past testing device 20.

After sufficient time to allow any remaining reagent (i.e., the reagent that has not reacted with the analyte) to react with testing device 20, an operator (or machine) may read the test result in window 23, through which testing device 20 is in view. The reaction time depends upon many factors, including the type of analyte and/or reagent. Alternatively, window 23 may be in any suitable position on apparatus 10. Of course, window 23 may need to be by testing device 20 in order for testing device 20 to be visible in window 23.

Testing device 20 provides a visual indicium of whether the analyte is present in the sample of material collected with sample acquisition device 24, and in some embodiments, the test result indicates the quantity of analyte. In the first exemplary embodiment, testing device 20 is a colorimetric sensor, which may include, for example, a polydiacetylene material, as described in U.S. Patent Application Publication No. 2004/0132217 A1, and U.S. Patent Application Serial No. 60/636,993, filed on December 17, 2004, both entitled, "COLORIMETRIC SENSORS CONSTRUCTED OF DIACETYLENE MATERIALS".

In the exemplary embodiment, a color of testing device 20 corresponds to a color-coding scheme. Testing device 20 may or may not provide a color change, depending upon whether the analyte is present in the sample of material. A user may view this color change through window 23. The color change may also be graded in order to indicate the quantity of analyte present. The quantity of analyte may, for example, be indicated by a color gradient which corresponds to "low level", "medium level", or "high level" indications. In some embodiments, apparatus 10 includes a label that illustrates the color-coding scheme, and an operator may compare the resulting color in window 23 with the label. In other embodiments, the color change cannot be detected with a human eye. In that case, a machine or electronic reader, such as a spectrometer, is used to detect the color change. In alternate embodiments, other testing devices may be used. For example, apparatus 10 may incorporate a testing device whose indicium of a test result is characterized by a pH change, or some other change in the characteristic of the medium being analyzed.

While FIGS. 1 and 3A show first exemplary apparatus 10 that is configured to receive only one sample acquisition assembly, apparatus 10 may be modified to receive more than one sample acquisition assembly. FIG. 3B shows a schematic illustration of an alternate embodiment of apparatus. As shown, apparatus 100 is configured to receive a first sample acquisition assembly 102 and a second sample acquisition assembly 104. Apparatus 100 includes first chamber 106 and second chamber 108, which are separated from one another. First chamber 106 is configured to receive eluted sample 110 from first sample acquisition assembly 102, while second chamber 108 is configured to receive eluted sample 112 from second sample acquisition assembly 104. More than one chamber 106 and 108 may be necessary to separate the samples that are eluted from each sample acquisition assembly 102 and 104. Apparatus 100 includes a separate channel and testing device (not shown) in fluidic communication with each chamber 106 and 108, where each channel and testing device may be similar to channel 21 and testing device 20 illustrated in FIG. 1. Each testing device may be adapted to detect a different analyte. Apparatus 100 may be used to detect more than one analyte in a sample of material, and the detection may be essentially simultaneous.

FIG. 4 is a second exemplary and illustrative configuration of an apparatus 60. Apparatus assembly 60 includes base 62 and indicator cap 66. Base 62 includes a first end 62A and a second end 62B, which are positioned on opposite sides of base 62. First end 62A of base 62 is configured to interchangeably receive indicator cap 66 and a sample acquisition assembly 64. Second end 62B of base 62 includes bottom surface 63 configured to support base 62 in a generally upright orientation relative to a generally horizontal surface on which base 62 is placed. Timer 61 may be attached to base 62. Timer 61 may assist an apparatus operator in timing a reaction time between a reagent and analyte, which will be discussed below. In alternate embodiments, apparatus assembly 60 does not include a timer. In those instances, an operator may provide its own timer.

As previously described, the sample acquisition assembly 64 includes a sample acquisition device 68 which may be any suitable device or assembly. In the illustrated embodiment, sample acquisition device 68 is similar to sample acquisition device 24 of FIGS. 1 and 3A. In particular, sample acquisition device 68 is a swab including hollow shaft 70 having proximal end 70A and distal end 70B, with porous medium 72 attached to distal end 70B of hollow shaft 70. Porous medium 72 is positioned over distal end 70B of hollow shaft 70, which includes at least one opening for allowing fluid 76 to move through second end 70B of hollow shaft 70, and contact porous medium 72.

The sample acquisition assembly 64 includes fluid reservoir 74. Fluid resevoir 74 is positioned in selective fluidic communication with first end 70A of hollow shaft 70 of sample acquisition device 68. Fluid reservoir 74 retains fluid 76, which elutes the sample of material from porous medium 72 of sample acquisition device 68 after fluid 76 is released from fluid reservoir 74.

In FIG. 4, fluid reservoir 74 is a syringe, where fluid 76 is retained in fluid reservoir 74 and plunger member 74A may be depressed in order to release fluid 76 from fluid reservoir 74. FIG. 5 illustrates an alternate embodiment of a sample acquisition assembly 80 for the apparatus of FIG. 4, where fluid reservoir 74 has been replaced with fluid reservoir 75. In the alternate embodiment shown in FIG. 5, fluid reservoir 75 is a deformable squeeze bulb with a break-off nib 77 to control the fluid 78 flow out of fluid reservoir 75. Note that as illustrated in FIG. 5, sample acquisition assembly 80 is not fully introduced into opening 82 of base 62.

In other alternate embodiments, fluid reservoir 74 is an accordion pleat bulb, or another type of reservoir that can selectively release fluid with greater pressure than a deformable squeeze bulb. A greater amount of pressure may be desirable in order to elute more of the sample of material from the porous medium 72 of sample acquisition device 68. Certain analytes may be more sensitive and a greater quantity of analyte may be required in order to be detected by the testing device. In those cases, it is preferred that a syringe or other device that is capable of releasing fluid with more pressure is used to release fluid 76, which elutes the sample of material from the porous medium 72.

Returning now to FIG. 4, indicator cap 66 includes projection 66A, which is configured to fit within opening 82 that is positioned at first end 62A of base 62, thereby allowing the attachment of indicator cap 66 to base 62. Of course, indicator cap 66 is not attached to base 62 until after sample acquisition assembly 64 has been removed from base 62. Bottom portion (or "underside") 66B of indicator cap 66 is configured to position base 62 in a generally upright orientation relative to a generally horizontal surface on which indicator cap 66 is placed. In this way, indicator cap 66 and bottom surface 63 of base 62 share a similar function of supporting base 62 relative to a generally horizontal surface.

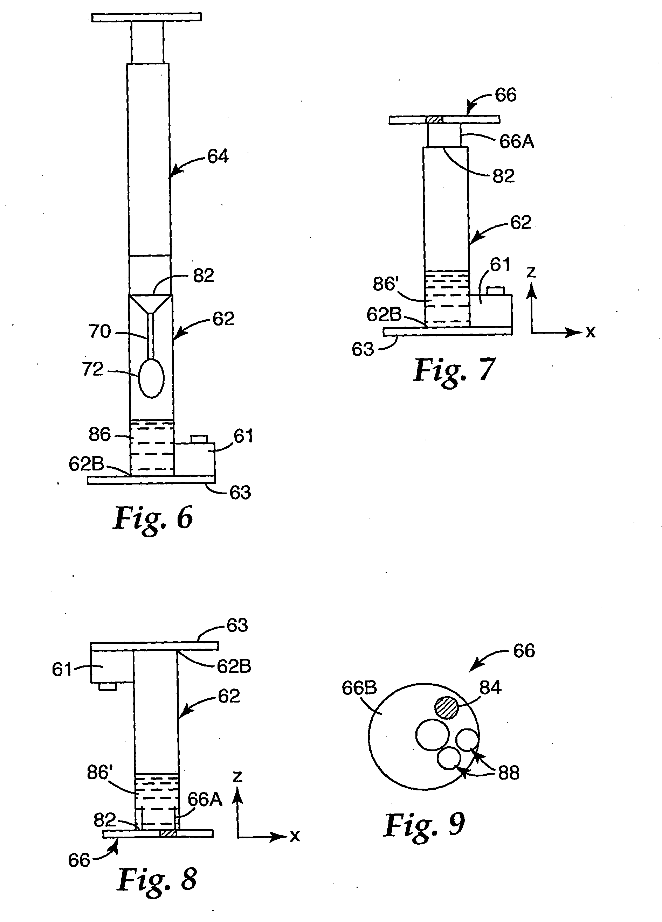

Indicator cap 66 includes a channel and testing device (not shown) similar to channel 21 and testing device 20 of FIGS. 1 and 3A. Preferably, the testing device provides a visual indicium of a test result, which is visible through window 84 (shown in FIG. 9, which is a view of underside 66B indicator cap 66). In the exemplary embodiment, the analyte is detected by an indirect assay, and the testing device is a colorimetric sensor. The colorimetric sensor may be similar to the colorimetric sensor described above in reference to testing device 20 of FIGS. 1 and 3A. Rather than positioning channel 21 and testing device 20 in a downward slant as in apparatus 10', indicator cap 66 includes an absorbent material to create a wicking action to encourage a fluid flow past the testing device. This will be described in reference to FIG. 8 below.

The present invention may be used to detect an analyte in a sample of material. During the sample preparation stage of testing a sample of material, when base 62 is in a sample preparation orientation (shown in FIG. 4), a sample of material is eluted from the sample acquisition device 68. A sample of material is collected using sample acquisition device 68. Hollow shaft 70 is handled to contact porous medium 72 with a sample source, thereby adhering a sample of material to porous medium 72. After the sample is collected, sample acquisition assembly 64 may be introduced into opening 82 (shown in FIG. 5) of base 62. Plunger member 74 as shown in FIG. 4 may be depressed or nib 77 broken in order to release fluid 76 or 78 from fluid reservoir 74 or 75 and introduce fluid 76 or 78 into hollow shaft 70 of sample acquisition device 68. Fluid 76 flows through hollow shaft 70 and contacts porous medium 72 of sample acquisition device 68. As fluid 76 moves through porous medium 72, at least some of the sample of material from porous medium 72 is eluted, thereby rendering "eluted sample" 86 (shown in FIG. 6).

Eluted sample 86 moves through base 62 in a first flow path, which is formed by base 62 in its sample preparation orientation. As FIG. 6 shows, eluted sample 86' is retained in base 62 (which forms a fluid chamber). As previously discussed, eluted sample 86 is mixed with a reagent while base 62 is in its sample preparation orientation, resulting in eluted sample 86' shown in FIG. 7. The reagent and eluted sample 86 may require a minimum amount of time to react. The reaction time may be monitored by timer 61, which may include a preset countdown timer or an operator may set the timer. As with the first exemplary embodiment, an operator may agitate the reagent and eluted sample 86 to aid the reaction between the reagent and analyte. The agitation may occur by hand or with a machine, such as a vortex mixer.

After eluted sample 86' is retained in base 62, sample acquisition device 68 is removed from opening 82 of base 62 and indicator cap 66 may be attached to opening 82 of base 62. FIG. 7 shows base 62 (still in its sample preparation orientation) and indicator cap 66, where projection 66A of indicator cap 66 is aligned with opening 82 of base 62.

As FIGS. 7-8 illustrate, projection 66A of indicator cap 66 is configured to fit within opening 82. After indicator cap 66 is attached to base 62, base 62 may be moved from its sample preparation orientation to its testing orientation, which is shown in FIG. 8. Specifically, base 62 is rotated about 180°, so that second end 62B of base 62 has a greater z-coordinate than first end 62A (see orthogonal x-z coordinates in FIGS. 7 and 8). In the testing orientation, indicator cap 66 supports base 62. In the sample preparation orientation, it is unlikely that eluted sample 86' disposed in base 62 will contact indicator cap 66, and therefore, the testing stage typically cannot begin until base 62 is in its testing orientation. Because the apparatus operator may manually move base 62 between its sample preparation orientation to its testing orientation, the present invention allows the operator to control when the testing stage begins.

After base 62 is moved into its testing orientation, eluted sample 86' begins moving through the channel (not shown in FIG. 8) in indicator cap 66, where the channel provides a flow path for fluid to flow to the testing device (not shown in FIG. 8). The testing device is positioned between the channel and the absorbent material, and the absorbent material (not shown) helps to create fluid flow past the testing device by a wicking action.

FIG. 9 shows an underside 66B of indicator cap 66, which includes window 84, and color-code diagram 88. After eluted fluid 86' flows past the testing device disposed in indicator cap 66 and reacts therewith, a user may observe the test result in window 84. Similar to window 23 of FIG. 3A, a colorimetric sensor (or other testing device) is visible through window 84. The operator may then compare the color that appears in window 84 with color-code diagram 88 to interpret the test result. Each circle in color-code diagram 88 may be a different color or a different intensity of a color hue, where each color or hue represents a different test result. Thus, the operator may choose the circle in color-code diagram 88 that is closest to the color appearing in window 84 in order to determine the test result. If another testing device is used, which for example outputs a symbol or pH, color-code diagram 88 may be modified accordingly to show what each symbol, pH, or other indicium represents.

Although underside 66B of indicator cap 66, window 84, and color-code diagram 88 are shown in FIG. 8 to be circular, indicator cap 66, window 84, and color-code diagram 88 may be any suitable shape. Furthermore, in alternate embodiments, window 84 and/or the color-code diagram 88 may be in another suitable position on indicator cap 66 or base 62.

As with first exemplary apparatus 10 of FIGS. 1 and 3 A, apparatus assembly 60 may be modified to accommodate the testing of more than one sample of material at a time, and/or the detection of more than one analyte at a time.

Although the present invention has been described with reference to preferred embodiments, workers skilled in the art will recognize that changes may be made in form and detail without departing from the spirit and scope of the invention.

The complete disclosures of the patents, patent documents and publications cited herein are incorporated by reference in their entirety as if each were individually incorporated. Various modifications and alterations to this invention will become apparent to those skilled in the art without departing from the scope and spirit of this invention. It should be understood that this invention is not intended to be unduly limited by the illustrative embodiments and examples set forth herein and that such examples and embodiments are presented by way of example only with the scope of the invention intended to be limited only by the claims set forth herein as follows.

## Claims

1. An apparatus for detecting an analyte in a sample of material, the apparatus comprising:
a housing (14) including an opening configured to receive a sample acquisition device (24) comprising the sample of material;
a chamber (16) configured to receive an eluted sample from the sample acquisition device;
a testing chamber including a testing device (20) adapted to detect the analyte; and
a continuously unobstructed passageway (21) connecting the chamber configured to receive the eluted sample with the testing chamber and being oriented to provide fluid flow from the first chamber to the testing chamber when the apparatus is in a first orientation and to restrict fluid flow to the testing chamber when the apparatus is in another orientation, wherein the testing device comprises a colorimetric sensor for providing a visual indicium of a test result;
wherein the first chamber comprises a reagent disposed therein;
wherein the testing chamber is in fluidic connection with the chamber configured to receive a sample;
wherein the testing chamber is between the chamber configured to receive the eluted sample and a chamber having an absorbent material (22).

2. The apparatus of claim 1, wherein the apparatus further comprises first (106) and second (108) chambers configured to receive eluted samples and first and second testing chambers including first and second testing devices and each of the first and second chambers is connected to the first and second testing chambers via separate channels orientated to provide fluid flow to the first and second testing chambers when the apparatus is in the first orientation and to restrict fluid flow to the first and second testing chambers when the apparatus is in another orientation.

3. The apparatus of claim 1 or claim 2 in combination with a sample acquisition device comprising:
a hollow shaft having a porous medium at a distal end of the hollow shaft; and
a fluid reservoir coupleable to a proximal end of the hollow shaft to provide fluid flow to elute the sample of material.

4. The apparatus of claim 1 wherein a flow path along the passageway to the testing chamber is sloped.

5. A method of detecting an analyte in a sample of material, the method comprising:
eluting a sample of material from a sample acquisition device into the chamber configured to receive an eluted sample in the apparatus of claim 1;
combining the eluted sample with a reagent; and
rotating the apparatus at least 90 degrees and testing the eluted sample using the testing device in the apparatus.

## Patentansprüche

1. Apparat zum Detektieren eines Analyten in einer Materialprobe, wobei der Apparat Folgendes umfasst:
ein Gehäuse (14), das eine Öffnung enthält, die dafür konfiguriert ist, eine Probenerfassungsvorrichtung (24) aufzunehmen, welche die Materialprobe umfasst;
eine Kammer (16), die dafür konfiguriert ist, eine eluierte Probe aus der Probenerfassungsvorrichtung aufzunehmen;
eine Testkammer, die eine Testvorrichtung (20) enthält, die dafür ausgelegt ist, den Analyten zu detektieren; und
einen durchgehend unbehinderten Durchgang (21), der die Kammer, die dafür konfiguriert ist, die eluierte Probe aufzunehmen, mit der Testkammer verbindet und so ausgerichtet ist, dass er einen Fluidfluss von der ersten Kammer zu der Testkammer ermöglicht, wenn sich der Apparat in einer ersten Ausrichtung befindet, und den Fluidfluss zu der Testkammer unterbindet, wenn sich der Apparat in einer anderen Ausrichtung befindet, wobei die Testvorrichtung einen kolorimetrischen Sensor umfasst, um eine sichtbare Anzeige eines Testergebnisses bereitzustellen;
wobei in der ersten Kammer ein Reagens angeordnet ist;
wobei die Testkammer in Strömungsverbindung mit der Kammer steht, die dafür konfiguriert ist, eine Probe aufzunehmen;
wobei sich die Testkammer zwischen der Kammer, die dafür konfiguriert ist, die eluierte Probe aufzunehmen, und einer Kammer, die ein Absorbensmaterial (22) enthält, befindet.

2. Apparat nach Anspruch 1, wobei der Apparat des Weiteren eine erste (106) und eine zweite (108) Kammer umfasst, die dafür konfiguriert sind, eluierte Proben aufzunehmen, und eine erste und eine zweite Testkammer umfasst, die eine erste und eine zweite Testvorrichtung enthalten, wobei sowohl die erste als auch die zweite Kammer mit der ersten und der zweiten Testkammer über separate Kanäle verbunden sind, die so ausgerichtet sind, dass sie einen Fluidfluss zu der ersten und der zweiten Testkammer ermöglichen, wenn sich der Apparat in der ersten Ausrichtung befindet, und den Fluidfluss zu der ersten und der zweiten Testkammer unterbinden, wenn sich der Apparat in einer anderen Ausrichtung befindet.

3. Apparat nach Anspruch 1 oder Anspruch 2 in Kombination mit einer Probenerfassungsvorrichtung, die Folgendes umfasst:
einen hohlen Schaft, der ein poröses Medium an einem distalen Ende des hohlen Schaftes aufweist; und
ein Fluidreservoir, das an ein proximales Ende des hohlen Schaftes gekoppelt werden kann, um einen Fluidfluss zum Eluieren der Materialprobe bereitzustellen.

4. Apparat nach Anspruch 1, wobei ein Strömungspfad entlang des Durchgangs zu der Testkammer ein Gefälle aufweist.

5. Verfahren zum Detektieren eines Analyten in einer Materialprobe, wobei das Verfahren Folgendes umfasst:
Eluieren einer Materialprobe aus einer Probenerfassungsvorrichtung in die Kammer, die dafür konfiguriert ist, eine eluierte Probe aufzunehmen, in dem Apparat nach Anspruch 1;
Zusammenbringen der eluierten Probe mit einem Reagens; und
Drehen des Apparates um mindestens 90 Grad und Testen der eluierten Probe mittels der Testvorrichtung in dem Apparat.

## Revendications

1. Appareil de détection d'un analyte dans un échantillon de matériau, l'appareil comprenant :
un boîtier (14) qui comprend une ouverture configurée pour recevoir un dispositif (24) d'acquisition d'échantillons qui contient un échantillon de matériau,
une chambre (16) configurée pour recevoir et éluer l'échantillon du dispositif d'acquisition d'échantillon,
une chambre de test qui comprend un dispositif de test (20) adapté pour détecter l'analyte et
un passage (21) exempt d'obstruction sur toute sa longueur, qui relie la chambre configurée pour recevoir l'échantillon élué à la chambre de test et est orienté de manière à délivrer un écoulement de fluide de la première chambre à la chambre de test lorsque l'appareil se trouve dans une première orientation et à restreindre l'écoulement de fluide vers la chambre de test lorsque l'appareil est dans une autre orientation, le dispositif de test comprenant un détecteur colorimétrique qui donne une indication visuelle du résultat d'un test,
un réactif étant disposé dans la première chambre,
la chambre de test étant en communication d'écoulement avec la chambre configurée pour recevoir un échantillon,
la chambre de test étant située entre la chambre configurée pour recevoir l'échantillon élué et une chambre qui présente un matériau absorbant (22).

2. Appareil selon la revendication 1, qui comprend en outre une première chambre (106) et une deuxième chambre (108) configurées pour recevoir des échantillons élués, ladite première et ladite deuxième chambre de test comprenant un premier et un deuxième dispositif de test, chacune parmi la première et la deuxième chambre étant reliée à la première et à la deuxième chambre par des canaux séparés orientés de manière à assurer un écoulement de fluide vers la première et la deuxième chambre de test lorsque l'appareil se trouve dans la première orientation et à restreindre l'écoulement de fluide vers la première et la deuxième chambre de test lorsque l'appareil se trouve dans une autre orientation.

3. Appareil selon la revendication 1 ou la revendication 2, combiné avec un dispositif d'acquisition d'échantillons comprenant :
une tige creuse dotée d'un milieu poreux à son extrémité distale et
un réservoir à fluide qui peut être relié à l'extrémité proximale de la tige creuse pour assurer un écoulement de fluide en vue d'éluer l'échantillon de matériau.

4. Appareil selon la revendication 1, dans lequel le parcours d'écoulement le long du passage vers la chambre de test est en pente.

5. Procédé de détection d'un analyte dans un matériau d'échantillons, le procédé comprenant les étapes qui consistent à :
éluer un échantillon de matériau provenant d'un dispositif d'acquisition d'échantillons dans une chambre configurée pour recevoir un échantillon élué dans l'appareil selon la revendication 1,
combiner l'échantillon élué et un réactif et
tourner l'appareil d'au moins 90 degrés et tester l'échantillon élué en utilisant le dispositif de test de l'appareil.
